# EUROPEAN PATENT SPECIFICATION

(11) **EP 3 271 845 B1**
(45) Date of publication and mention of the grant of the patent: **16.09.2020**
(21) Application number: 16711701.9
(22) Date of filing: 09.03.2016
(51) Int. Cl.: G16H 40/63, G06F 3/14, G06F 3/0488, G06F 3/147

(54) **INPUT UNIT, PATIENT MONITOR AND PATIENT MONITORING SYSTEM**
EINGABEEINHEIT, PATIENTENMONITOR UND PATIENTENÜBERWACHUNGSSYSTEM
UNITÉ D'ENTRÉE, DISPOSITIF DE SURVEILLANCE DE PATIENT ET SYSTÈME DE SURVEILLANCE DE PATIENT

(30) Priority: 18.03.2015 JP 2015054377
(43) Date of publication of application: 24.01.2018
(73) Proprietor: Nihon Kohden Corporation, Shinjyuku-ku Tokyo 161-8560 (JP)
(72) Inventor: SUGAWARA, Hironori, Tokyo 161-8560 (JP); KUBO, Hiroshi, Tokyo 161-8560 (JP)
(74) Representative: Hoffmann Eitle
(86) International application number: PCT/JP2016/001305
(87) International publication number: WO 2016/147617

(56) References cited:
- US-A1- 2001 037 366
- US-A1- 2014 067 007

## Description

The presently disclosed subject matter relates to an input unit, a patient monitor, and a patient monitoring system.

### Background Art

Related art patient monitors are configured to measure and display various vital signs (e.g., blood pressure, arterial oxygen saturation, body temperature, respiration, electrocardiogram, heart rate and the like). To access necessary information, a medical personnel operates hard keys, push switches, trim knobs and the like.

Patient monitors are used not only in medical wards but also in other various places such as operating rooms and ICUs. In a case of being used in operating rooms or ICUs, often times, a patient monitor is installed at a location where an operator (a medical staff) may find difficulty in reaching the patient monitor, but yet the operator needs to manually operate the patient monitor.

There are cases where an input unit is placed beside the patient. The input unit is provided in a separate housing from a patient monitor, and is sometimes provided with hard keys for operating the patient monitor. Although excellent in operability, functions are limited with the hard keys of the input unit.

US2013/0225952A1 discloses a system for measuring blood oxygen levels. The system has a display that is separately provided from a main unit of a measuring apparatus. The separate display displays vital sign waveforms and the like as displayed on the main unit (see, e.g., Fig. 1 of US2013/0225952A1). However, the separate display is not designed to be used for changing the settings of the measuring apparatus. Therefore, the operability in cases where the system is used in operating rooms and the like as described above cannot be improved.

In both of the examples described above (the example in which operations are made using an operation panel of the main unit and the example in which operations are made using the input unit), when detailed settings are being carried out, a setting screen is displayed on the display of the main unit. With this setting screen being displayed, measured values and vital sign waveforms obtained from a patient becomes difficult to be visually checked (or cannot be visually checked).

US 2001/037366 A1 (WEBB JAMES D[US] ET AL) 1 November 2001 describes a system for allowing medical data obtained at a local site to be transferred to a data processing system located at a remote site, according to the prior art.

US 2014/067007A1 (DREES SCOTT [US] ET AL) 6 March 2014 describes a method of visualizing a user interaction with a clinician programmer. A user engagement with respect to a screen of the clinician programmer is detected via one or more sensors associated with the screen of the clinician programmer. One or more locations on the screen of the clinician programmer corresponding to the user engagement is determined. An external monitor is communicatively coupled to the clinician programmer. The external monitor displays one or more cursors that graphically represent the one or more locations on the screen of the clinician programmer corresponding to the user engagement, respectively.

### Summary

The object of the invention is achieved by the subject-matter of the independent claims. Advantageous embodiments are defined in the dependent claims. Further examples are provided for facilitating the understanding of the invention.

Illustrative aspects of the present invention provide an input unit, a patient monitor, and a patient monitoring system in which settings of the patient monitor can be changed while ensuring the visibility of vital signs.

According to an illustrative aspect of the present invention, there is provided an input unit according to claim 1.

According to another illustrative aspect of the present invention, there is provided a patient monitor according to claim 9.

According to another illustrative aspect of the present invention, a patient monitoring system includes the input unit and the patient monitor described above.

### Brief Description of Drawings

[fig 1]Fig. 1 illustrates how a patient monitoring system according to an exemplary embodiment of the present invention is used.
[fig.2] Fig. 2 is a block diagram of a configuration of an input unit according to an exemplary embodiment of the present invention.
[fig.3]Fig. 3 is a block diagram of configuration of a patient monitor according to an exemplary embodiment of the present invention.
[fig.4]Fig 4 illustrates an example of a display screen to be displayed on a display and input section of the input unit.
[fig.5]Fig. 5 is a flowchart of an example of a series of processings starting from a transmission of setting information from the input unit.
[fig.6]Fig. 6 illustrates another example of a display screen to be displayed on a display and input section of the input unit.
[fig.7]Fig. 7 is a flowchart of an example of a display control by a controller of the patient monitor.
[fig.8]Fig. 8 illustrates an example of operations of the patient monitoring system.
[fig.9]Fig. 9 is a flowchart associated with the operations illustrated in Fig. 8.

### Description of Embodiments

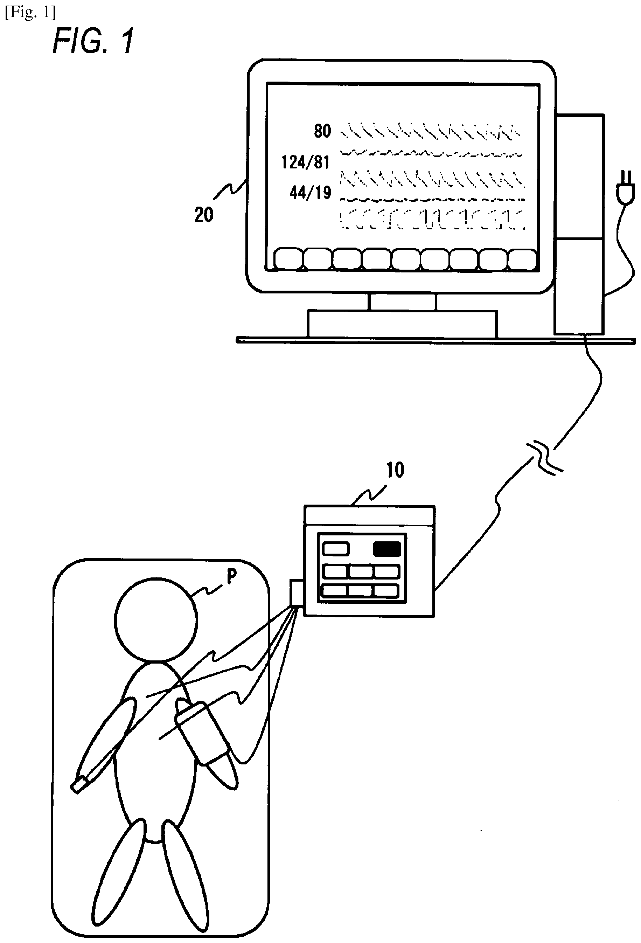

Hereinafter, exemplary embodiments of the present invention will be described with reference to the drawings. Fig. 1 illustrates how a patient monitoring system 1 according to an exemplary embodiment of the present invention is used. The patient monitoring system 1 includes an input unit 10 and a patient monitor 20.

The patient monitor 20 can be placed at a location separated from the input unit 10 and a patient P. For example, the patient monitor 20 may be hung from the ceiling of an operating room or placed on a shelf. The patient monitor 20 receives biological signals of the patient P through the input unit 10, and converts the signals to visible vital signs (e.g., waveforms and measurement values of various vital parameters). Then, the patient monitor 20 displays the vital signs, and performs notification by alarming.

While the installation place is not particularly limited, the input unit 10 is mainly installed at a position which is in the vicinity of the patient P, and in which the operator (medical staff) can easily operate the unit. The input unit 10 acquires biological signals from a sensor group (a cuff, a probe, and the like) attached respectively to portions of the patient P. The operator changes various settings of the patient monitor 20 by using an input interface (synonymous with a display and input section 12 which will be described later, preferably a touch panel) of the input unit 10. The input unit 10 transmits setting information to the patient monitor 20 connected to the input unit 10. Here, the setting information means information indicative of instructions for changing various settings of the patient monitor 20.

Also during periods when the input unit 10 is operated, and when the setting information transmitted from the input unit 10 is reflected, the vital signs (waveforms and measurement values of various parameters) of the patient P are continued to be displayed on the patient monitor 20. In the patient monitor 20, namely, the setting change is performed while the vital signs of the patient P are displayed.

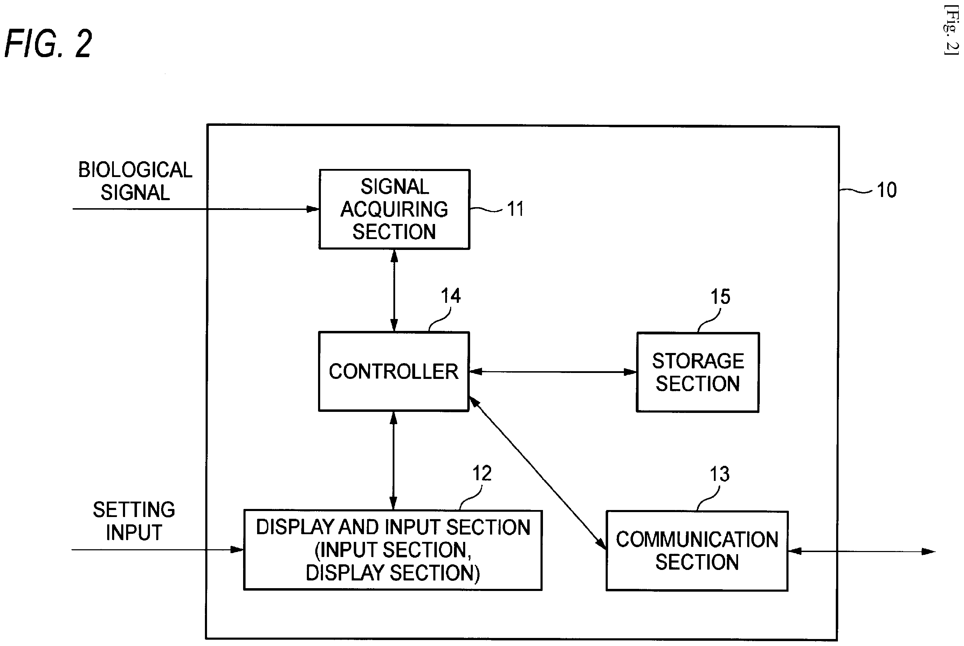

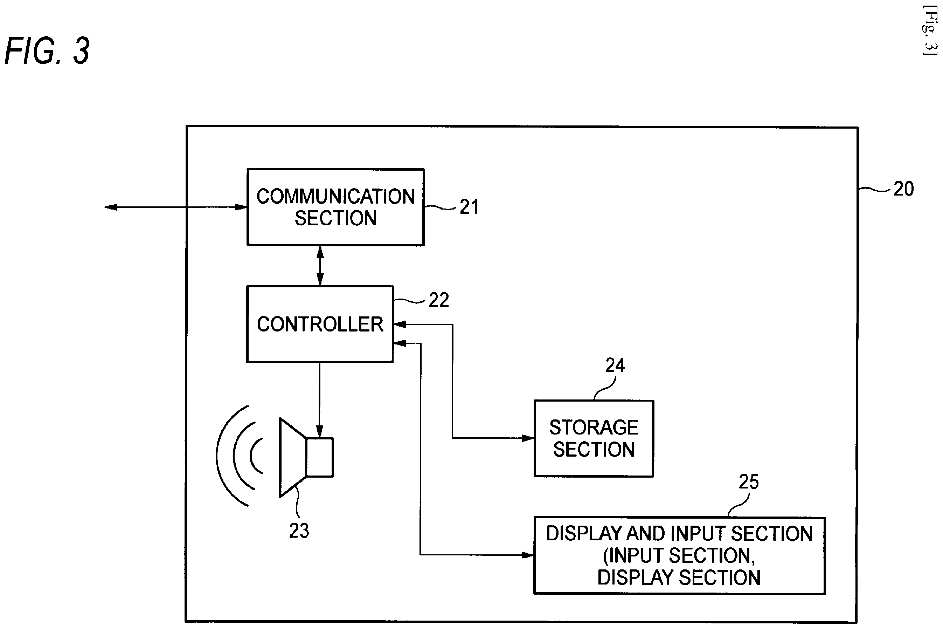

Operations and configurations of the patient monitoring system 1 will be described in more detail with reference to Figs. 2 and 3. Fig. 2 is a block diagram of an internal configuration of the input unit 10, and Fig. 3 is a block diagram of an internal configuration of the patient monitor 20.

Referring to Fig. 2, first, the input unit 10 will be described. The input unit 10 includes a signal acquiring section 11, the display and input section 12, a communication section 13, a controller 14, and a storage section 15.

The signal acquiring section 11 acquires biological signals (such as electrocardiogram signals and wave signals) of the patient P from the various sensors (e.g., disposable electrodes and an SpO2 probe) attached to the patient P. The various sensors attached to the patient P may be equivalent to those used with a general patient monitor.

The display and input section 12 functions as a display section to display information for the operator, and also as an input section to receive an input from the operator. For example, the display and input section 12 may be a general touch panel. The display and input section 12 mainly displays the setting screen for the patient monitor 20. The operator refers the setting screen, and performs setting of the patient monitor 20.

The communication section 13 transmits and receives data to and from the patient monitor 20. For example, the communication section 13 transmits operations performed on the display and input section 12 (i.e., setting information for the patient monitor 20) to the patient monitor 20. Moreover, the communication section 13 receives the current setting status transmitted from the patient monitor 20. The communication section 13 is configured by hardware (circuits and the like) and software on which a wired or wireless communication function is mounted. Namely, the input unit 10 and the patient monitor 20 are requested to transmit and receive data by means of wired or wireless communication. The communication process of the communication section 13 is controlled by the controller 14.

The controller 14 is a processor configured to control the input unit 10. The controller 14 reads out various programs from the storage section 15, and executes the programs to perform various controls on the input unit 10. Of course, a part of the control process may be implemented by hardware.

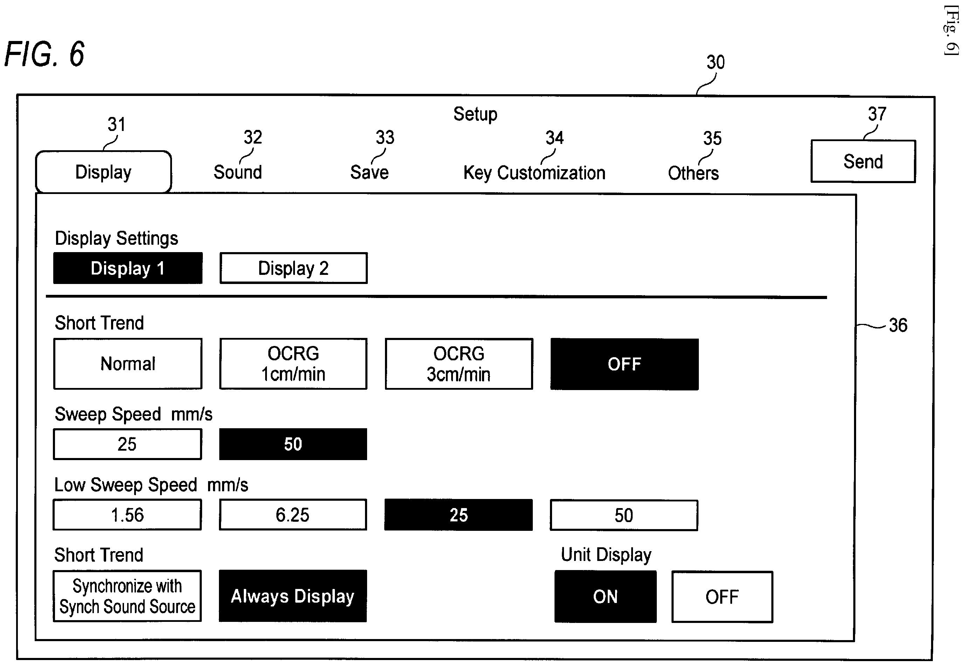

The control process of the controller 14 includes also the control of the display of the display and input section 12. When the communication section 13 receives the setting status from the patient monitor 20, the controller 14 can customize the display screen according to the setting status, and cause the screen to be displayed on the display and input section 12. An example of the display control will be described later with reference to Fig. 6.

The storage section 15 is a storage device which stores various data of the input unit 10. For example, the storage section 15 may be a hard disk drive, or a storage device which can be attached to and detached from the input unit 10 (such as a USB memory).

Then, the configuration of the patient monitor 20 will be described with reference to Fig. 3. The patient monitor 20 displays measurement values and waveforms of various vital parameters (the blood pressure, an electrocardiogram, the heart rate, a respiratory waveform, the body temperature, and the like) based on the biological signals which are input to the monitor through the input unit 10. The patient monitor 20 has a communication section 21, a controller 22, a speaker 23, a storage section 24, and a display and input section 25.

The communication section 21 transmits and receives data to and from the input unit 10. For example, the communication section 21 receives biological signals, the setting information, and the like from the input unit 10. As described above, the setting information means various sets of information for changing settings of the patient monitor 20. The communication section 21 transmits the current setting status (the contents of the settings) of the patient monitor 20 to the input unit 10. The communication section 21 is configured by hardware (circuits and the like) and software on which a wired or wireless communication function is mounted. The communication process of the communication section 21 is controlled by the controller 22.

The controller 22 is a processor configured to perform various controls of the patient monitor 20. The controller 22 reads out various programs from the storage section 24, and executes the programs to perform various controls on the patient monitor 20. It is a matter of course that a part of the control process may be implemented by hardware.

The control process of the controller 22 includes: conversion from the biological signals to various vital signs (measurement values and waveforms of various parameters); a display control on the patient monitor 20 (screen control on the display and input section 25); a setting process on the own monitor in accordance with the setting information supplied from the input unit 10; a setting process and measurement control on the controller 14 of the input unit 10; and the like. When any one of the various calculated vital signs has an abnormal value, the controller 22 outputs an alarm sound through the speaker 23.

The speaker 23 outputs various alarm and notification sounds in accordance with the control by the controller 22. The storage section 24 is a storage device which stores various data of the patient monitor 20. For example, the storage section 24 may be a hard disk drive incorporated in the patient monitor 20, or a storage device which can be attached to and detached from the patient monitor 20 (such as a USB memory).

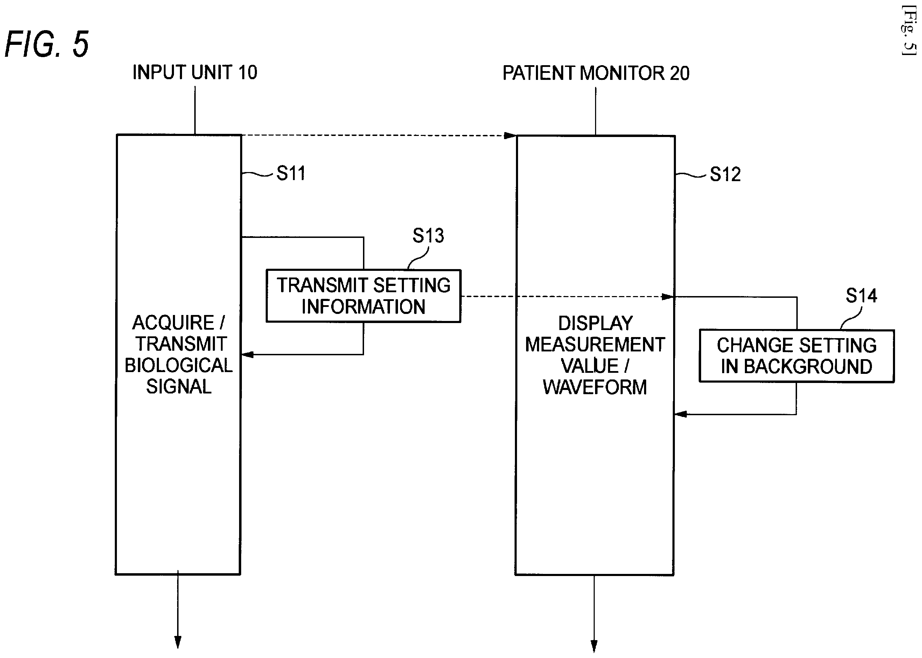

The display and input section 25 functions as a display section which displays information for the operator, and further functions as an input section which receives an input from the operator. For example, the display and input section 25 may be a general touch panel. The display and input section 25 mainly displays the measurement values and waveforms of various vital parameters of the patient P. As described above, the input unit 10 transmits the setting information of the patient monitor 20. Also during a period when a setting process according to the setting information transmitted from the input unit 10 is executed, the display and input section 25 displays the measurement values and waveforms of various vital parameters of the patient P. The display control will be described later with reference to Fig. 5.

Hereinafter, the display screens and operation controls of the input unit 10 and the patient monitor 20 will be described in more detail.

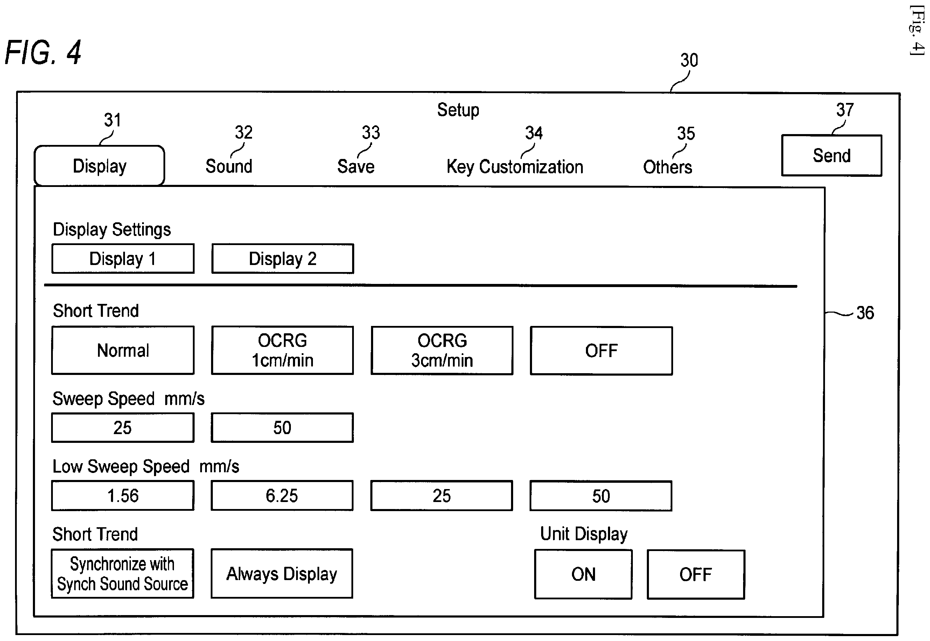

First, the basic operation of the patient monitoring system 1 will be described with reference to Fig. 4. Fig. 4 is a view showing an example of the display screen which is displayed on the display and input section 12 of the input unit 10. In Fig. 4, the display screen 30 is a setting screen for a basic setup of the patient monitor 20. In the display screen 30, superior tabs (a "Display" tab 31, a "Sound" tab 32, a "Save" tab 33, a "Key Customization" tab 34, and an "Others" tab 35) are disposed in an upper area of the screen. As described above, the input unit 10 is configured so as to display the setting screen for the patient monitor 20. Therefore, the storage section 15 of the input unit 10 stores a program which defines the setting screen for the patient monitor 20.

The operator touches one of the superior tabs with the finger to open the tab for setting. In the example, the "Display" tab 31 is selected, and therefore a display setting item screen 36 relating to the display of the patient monitor 20 is displayed. The operator selects a desired button in the display setting item screen 36 (e.g., "Display of unit = ON"), and then presses a transmission button 37. As a result, the communication section 13 transmits the setting information ("Display of unit = ON") to the patient monitor 20 through the controller 14.

The display screen 30 of Fig. 4 shows a mere example of the screen which is displayed on the display and input section 12, and which is the setting screen for the patient monitor 20. Moreover, a screen for setting display of various vital parameters (such as the SpO2 and an electrocardiogram), a setting screen for managing the patient, and the like may be displayed on the display and input section 12. A trigger for transmitting data is not limited to touching of the transmission button 37, and may be associated with another kind of operation.

The flow of a series of processes starting from transmission of the setting information from the input unit 10 will be described with reference to Fig. 5. The communication section 13 of the input unit 10 continuously transmits the biological signals acquired from the patient P, to the patient monitor 20 (S11). The communication section 21 of the patient monitor 20 receives the biological signals (S12). Then, the controller 22 converts the biological signals to vital sign waveforms (an electrocardiogram and the like) and measurement values (the blood pressure and the like), and displays them on the display and input section 25 (S12). The displaying process is continuously performed during a period when the patient P is set as the measurement object (S12).

When the instruction for transmitting setting information (the setting for the patient monitor 20, such as "Display of unit = ON") due to an operation on the display and input section 12 is issued, the communication section 13 of the input unit 10 transmits the setting information to the patient monitor 20 (S13). The communication section 21 of the patient monitor 20 receives the setting information in addition to the biological signals (S14). Then, the controller 22 changes the setting of the patient monitor 20 (own monitor) by using the received setting information (S14). At this time, the controller 22 continues the display of the vital sign waveforms (electrocardiogram and the like) and the measurement values (blood pressure and the like) (S12).

In this way, the setting of the patient monitor 20 is changed while the patient monitor 20 remains to display the vital sign waveforms (electrocardiogram and the like) and the measurement values (blood pressure and the like).

Fig. 5 shows the basic operations of the input unit 10 and the patient monitor 20, and various modifications may be made. Examples of modifications will be described below.

As a first modification, a configuration where the display and input section 12 of the input unit 10 can transmit setting information while checking the current setting status will be described. The controller 22 of the patient monitor 20 transmits the current setting status of the patient monitor 20 to the input unit 10 through the communication section 21. Here, the setting status is the currently settings of the patient monitor 20, and, for example, includes the following information.

[Display = [Short trend = OFF, Sweep speed = 50 ,,,,,,], Sound = [,,,,,,],,,,,,,

The setting status may be transmitted at given timings. For example, the transmission may be performed at a timing when the patient monitor 20 is powered ON, every hour, or at a timing when the setting change is completed.

The communication section 13 of the input unit 10 receives the setting status from the patient monitor 20. The received setting status of the patient monitor 20 is stored in the storage section 15 (data of the setting status which are stored in the storage section 15 are overwritten). The controller 14 reads out the setting status of the patient monitor 20 from the storage section 15, and produces a display screen which is to be displayed on the display and input section 12, in accordance with the setting status. Namely, the display and input section 12 displays a setting screen which is based on the setting status of the patient monitor 20. Fig. 6 shows an example of the display screen according to the setting status.

In Fig. 6, the currently set values are indicated in white characters. This allows the operator to change the setting while confirming the current settings of the patient monitor 20. The display screen of Fig. 6 is merely an example, and a different display screen may be displayed as long as the currently set values are displayed in a distinguished manner.

In response to operation of the operator, preferably, the color-inverted display is switched from the display of Fig. 6. In the case that, when "Display of unit = ON" is selected as shown in Fig. 6, the operator selects OFF, for example, it is preferable that the background of the interface of "ON" becomes white (characters are black), and the interface of "OFF" is switched to hollow characters.

Alternatively, the controller 22 of the patient monitor 20 may control the display of the display and input section 25 in accordance with the operated terminal (i.e., whether the display and input section 12 is operated or the display and input section 25 is operated). Hereinafter, the description will be made with emphasis on the control by the controller 22.

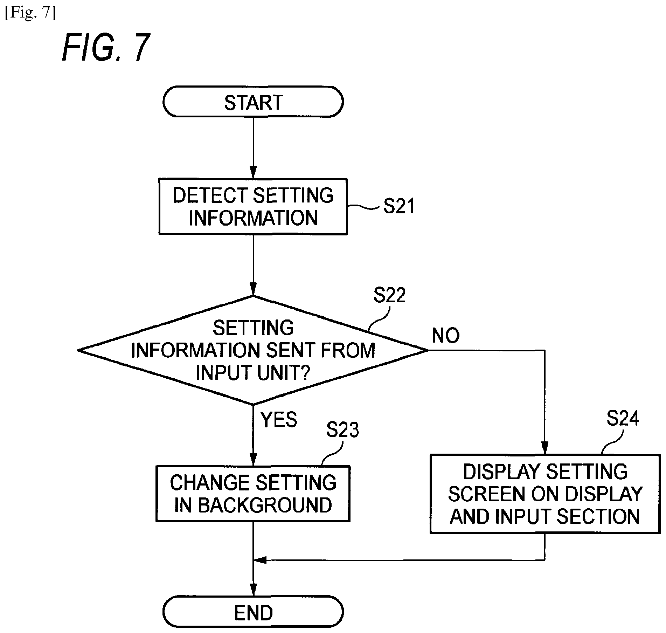

In this example, the operator may change also the setting of the patient monitor 20 by operating the display and input section 25. That is, the operator performs operations relating to the setting, through the display and input section 12 and also through the display and input section 25. Fig. 7 is a flowchart showing the display control by the controller 22 in the modification.

The controller 22 detects an input of setting information (S21). The setting information is input from the display and input section 25, or through the communication section 21 in response to an operation on the display and input section 12. The controller 22 determines whether the transmission source of the detected setting information is the input unit 10 or not (S22). Namely, the controller 22 determines whether the setting information is input by an operation on the display and input section 25 (or a remote operation controller for the patient monitor 20) or not (S22).

If the setting information is input from the input unit 10 (S22: Yes), the controller 22 changes the setting in the background while displaying the vital sign waveforms (electrocardiogram and the like) and the measurement values (blood pressure and the like) in a manner similarly to the control (S21, S14) shown in Fig. 5 (S23).

If the setting information is not input from the input unit 10, or in other words the information is input by manually operating the display and input section 25 (S22: No), the controller 22 causes a display screen corresponding to the setting screen (Fig. 4) to be displayed on the display and input section 25 so that the display screen is superimposed on the display screen which displays the vital sign waveforms (electrocardiogram and the like) and the measurement values (blood pressure and the like) (S24).

In the case that the display and input section 25 is operated as described above, the operator is in close proximity to the patient monitor 20. When the setting screen is displayed on the display and input section 25 and the setting is quickly performed, therefore, the easiness of the setting change can be ensured.

According to another modified example, a cursor display on the display and input section 12 and a cursor display on the display and input section 25 are synchronously moved.

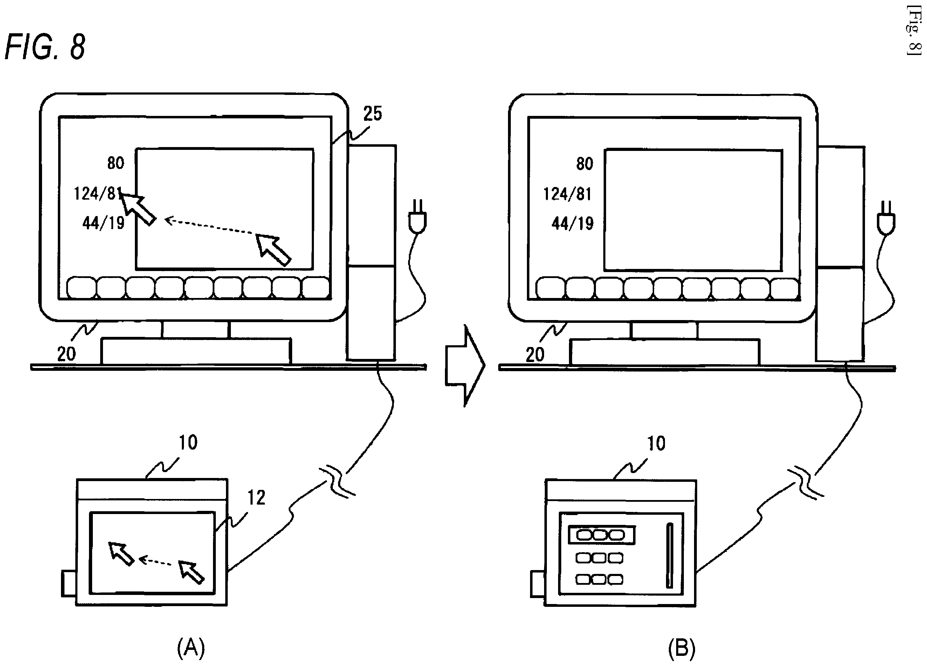

First, the operation image of this example will be described with reference to Fig. 8. Referring to (A) of Fig. 8, the operator operates the display and input section 12 of the input unit 10 to display a mouse cursor on the display and input section 12. In synchronization with the display, a mouse cursor is displayed on the display and input section 25 of the patient monitor 20 at a corresponding position (position corresponding to the operation of the mouse cursor on the display and input section 12). At the timing when the display of the mouse cursor is started, for example, the mouse cursor which is to be displayed on the display and input section 25 may be displayed at the coordinates of the center of the screen. While referring to the display and input section 25 on the patient monitor 20, the operator operates the display and input section 12 to move the position of the mouse cursor. The mouse cursor on the display and input section 12, and that on the display and input section 25 are moved in synchronization with each other.

The position of the mouse cursor which is displayed on the display and input section 25 may be set in accordance with a relative moving distance of the mouse cursor which is displayed on the display and input section 12. For example, the position of the finger which is placed on the display and input section 12 may be set as the starting point, and the position of the mouse cursor on the display and input section 25 may be moved in accordance with the moving distance by which the finger is moved from the starting point. That is, the mouse cursor on the display and input section 25 is requested to be displayed so as to correspond to the operation applied to the display and input section 12.

It is assumed that the operator double-clicks (performs the specific operation on) the display and input section 12 in a state where the mouse cursor is placed on the measurement value of the blood pressure in the display and input section 25. In accordance with the operation, the screen display is switched to the state of (B) of Fig. 8. In (B) of Fig. 8, the display and input section 25 of the patient monitor 20 displays measurement values of vital signs and vital sign waveforms. On the other hand, a setting screen related to the blood pressure (a display screen related to the click position) is displayed on the display and input section 12 of the input unit 10.

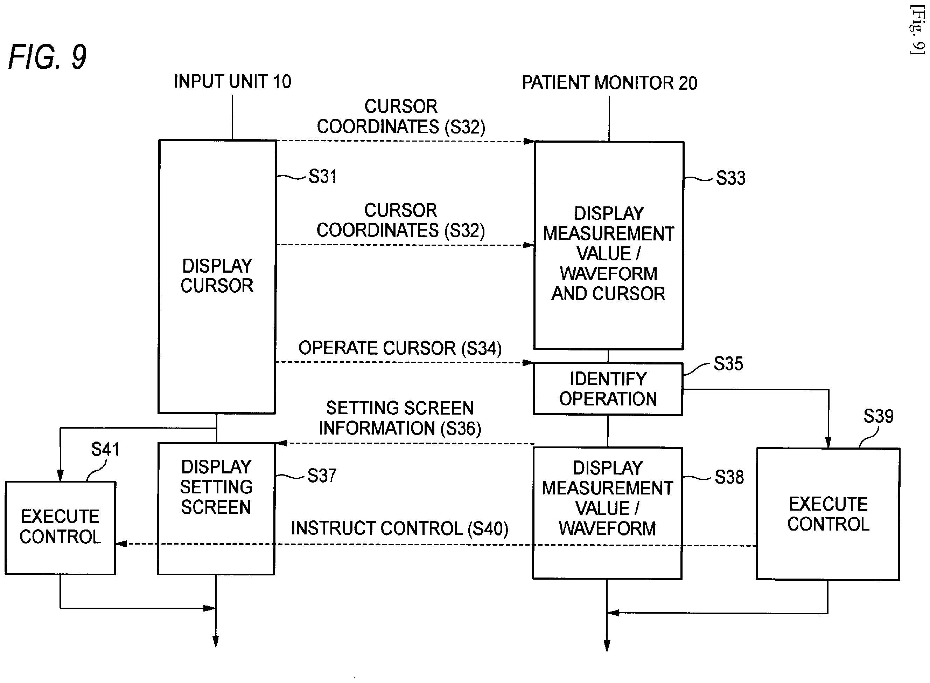

Referring to Fig. 9, an example of execution of the operation shown in Fig. 8 will be described. In accordance with pressing or the like of a specific button which is performed by the operator, first, the controller 14 causes the mouse cursor to be displayed on the display and input section 12 (S31). Furthermore, the controller 14 notifies the patient monitor 20 through the communication section 13 that the display of the mouse cursor is started (S32).

In response to the notification, the controller 22 causes the mouse cursor to be displayed in a superimposed manner on the screen displaying measurement values and waveforms (S33). Each time when the position of the mouse cursor is moved, the controller 14 detects cursor operation information (coordinates and the like). Then, the communication section 13 transmits the cursor operation information to the patient monitor 20 (S32). In accordance with the received cursor operation information (coordinates and the like), then, the controller 22 causes the mouse cursor to be displayed at the corresponding position on the display and input section 25 (S33).

In the case that the specific operation is performed (e.g., a double-click is performed), the controller 14 notifies of the operation of the mouse cursor through the communication section 13 (S34). The controller 22 receives the notification through the communication section 21. Based on the display position of the mouse cursor on the display and input section 25 at the timing when the specific operation is performed, the controller 22 identifies the kind of the operation (S35). This identification may be performed based on the relationship of the contents and coordinates of the display on the display and input section 25. In the case that a certain kind of setting (setting related to vital parameters, that related to the patient P, or the like) is designated, the controller 22 transmits information of the setting screen (such as information indicative of the kind of setting screen to be displayed, the information is referred to also as setting screen information), to the input unit 10 through the communication section 21 (S36). The controller 14 receives information of the setting screen through the communication section 13. In accordance with the received information of the setting screen, the controller 14 causes the setting screen to be displayed on the display and input section 12 (S37). On the display and input section 25, then, regular measurement values and vital sign waveforms continue to be displayed (S38).

By contrast, in the case that a certain control in the patient monitor 20 is designated (e.g., the start of the measurement of the NIBP or the like is instructed), the controller 22 executes the designated control (S39). In the case that also the control on the side of the input unit 10 is necessary (e.g., the pressure of a cuff attached to the patient P is raised through the input unit 10), the controller 22 notifies the control contents to the input unit 10 (S40). The controller 14 performs a control in accordance with the notification contents (S41).

As described above, the displays of the mouse cursor are synchronized with each other. Even when the operator is at a position that is separate from the patient monitor 20, therefore, the operator can easily operate the mouse cursor while viewing the screen on which waveforms are displayed.

Furthermore, the setting screen corresponding to the coordinates of the operating position of the mouse cursor is displayed on the display and input section 12 as described above. This enables the operator to operate the input unit 10 in feeling as if the operator touches the screen of the patient monitor 20. Of course, the synchronization may be applied to a cursor other than the mouse cursor, or another display effect.

Next, advantages of the patient monitoring system 1 according to the exemplary embodiment will be described. The input unit 10 has the display and input section 12 configured to display the setting screen for the patient monitor 20 and to receive an input. The communication section 13 transmits setting information input through the display and input section 12 to the patient monitor 20. According to the configuration, it is not necessary to display the setting screen on the display and input section 25 of the patient monitor 20, and the setting can be changed while ensuring the visibility of the patient monitor 20.

Moreover, with the input section and the display section being integrated as the display and input section 12, an operator can more easily carry out the settings.

As for the patient monitor 20, the patient monitor 20 changes the setting in background by using the setting information input from the input unit 10. That is, the patient monitor 20 changes the setting in background while continuing to display measurement values and vital sign waveforms. Therefore, the operator (medical staff) can quickly change the setting while keeping an eye on the condition of the patient P.

While the present invention has been described with reference to certain exemplary embodiments thereof, the scope of the present invention is not limited to the exemplary embodiments described above, and it will be understood by those skilled in the art that various changes and modifications may be made therein without departing from the scope of the present invention as defined by the appended claims.

In the above-described configuration, the display and input section 12 functions while integrating an input section with a display section. Even in a configuration where an input section (e.g., a mouse or a keyboard) and a display section (displaying device) are disposed separately from each other, however, the setting can be changed while ensuring the visibility of the patient monitor 20. In the above-described configuration, the display and input section 25 functions while integrating an input section with a display section. Alternatively, the patient monitor 20 may be configured so that an input section (e.g., a mouse or a keyboard) and a display section (displaying device) are disposed separately from each other.

A configuration where the patient monitor 20 directly acquires biological signals from the patient P may be employed. Namely, the patient monitor 20 may be configured so as to have cables which are to be connected to various sensors (e.g., an SpO2 probe and electrocardiogram electrodes).

A part of the above-described processes (the display control of the display and input section 12, and the like) of the controller 14 and the communication section 13 may be realized as a computer program which operates in the input unit 10. Similarly, a part of the processes of the controller 22 (the display control of the display and input section 25, and the like) and the communication section 21 may be realized as a computer program which operates in the patient monitor 20.

The programs may be stored in a non-transitory computer readable medium of any one of various types, and then supplied to the computer. The non-transitory computer readable medium includes tangible storage media of various types. Examples of the non-transitory computer readable medium are a magnetic recording medium (e.g., a flexible disk, a magnetic tape, and a hard disk drive), a magneto-optical recording medium (e.g., a magneto-optical disk), a CD-read only memory (ROM), a CD-R, a CD-R/W, a semiconductor memory (e.g., a mask ROM, a programmable ROM (PROM), an erasable PROM (EPROM), a flash ROM, and a random access memory(RAM)). Alternatively, the programs may be supplied to the computer by means of a transitory computer readable medium of any one of various types. Examples of the transitory computer readable medium are an electrical signal, an optical signal, and an electromagnetic wave. The transitory computer readable medium can supply the programs to the computer through a wired communication path such as a metal wire or an optical fiber, or a wireless communication path.

## Claims

1. An input unit (10) comprising:
a display section (12) configured to display a setting screen for changing a setting of a patient monitor (20) connected to the input unit (10);
an input section (12) configured to receive an input of setting information indicative of an instruction for changing the setting of the patient monitor; and
a communication section (13) configured to transmit a biological signal of a patient (P), acquired from a sensor attached to the patient (P), to the patient monitor (20) to allow the patient monitor (20) to display vital sign information of the patient (P) and also to transmit the setting information that is input from the input section (12) to the patient monitor (20) when an instruction for transmitting setting information due to an operation on the display and input section (12) is issued;
wherein the communication section (13) is configured to receive a current setting status from the patient monitor (20), and
wherein the display section (12) is configured to display the setting screen for the patient monitor (20), the setting screen being based on the received setting status.

2. The input unit (10) according to claim 1, wherein the display section (12) and the input section (12) are integrated as a touch panel.

3. The input unit (10) according to claim 1, further comprising a storage section (15) configured to store the setting status received by the communication section (13).

4. The input unit (10) according to claim 2, wherein the touch panel is configured to display a cursor, and
wherein the communication section (13) is configured to transmit information on an operation of the cursor to the patient monitor (20).

5. The input unit (10) according to claim 4, wherein the communication section (13) is configured to transmit coordinates, to which the cursor is operated on the touch panel, to the patient monitor (20), and to receive setting screen information corresponding to the coordinates from the patient monitor (20), and
the display section (12) is configured to display the setting screen corresponding to the setting screen information.

6. The input unit (10) according to claim 1, wherein the communication section (13) is configured to transmit the setting information that is input from the input section (12) to the patient monitor (20) to change the setting of the patient monitor (20) in a state in which the vital sign information is displayed on the patient monitor (20).

7. The input unit (10) according to claim 1, further comprising a storage section (15) storing a program defining the setting screen for the patient monitor (20).

8. The input unit (10) according to claim 1, wherein the display section (12) is configured to display the setting screen such that currently set values are distinguishable from other values.

9. A patient monitor (20) comprising:
a communication section (21) configured to receive a biological signal of a patient (P) and setting information from the input unit (10) of claim 1, the setting information being indicative of an instruction for changing a setting of the patient monitor (20);
a display section (25) configured to display vital sign information of the patient (P); and
a controller (22) configured to convert the biological signal to the vital sign information to control a display on the display section (25), and to change, upon receipt of the setting information, a setting of the patient monitor (20) in a state in which the vital sign information is displayed on the display section (25), wherein the communication section (21) is configured to transmit a current setting status to the input unit (10);
wherein the patient monitor (20) is configured to change the setting in background.

10. The patient monitor (20) according to claim 9, wherein the controller (22) displays, upon receipt of an input of cursor operation information from the input unit (10), a cursor at a corresponding position on the display section (25) based on the cursor operation information.

11. The patient monitor (20) according to any one of claims 9 to 10, further comprising an input section (25) configured to receive an input from an operator,
wherein the controller (22) is configured to display, upon receipt of the setting information from the input section (25), the vital sign information and a setting screen in a superimposed manner on the display section (25).

12. The patient monitor according to claim 9, wherein the display section (25) is configured to display the vital sign information including a vital sign waveform and a measurement value.

13. A patient monitoring system (1) comprising an input unit (10) and a patient monitor (20),
wherein the input unit (10) comprises:
a first display section (12) configured to display a setting screen for changing a setting of the patient monitor (20) connected to the input unit (10);
an input section (12) configured to receive an input of setting information indicative of an instruction for changing the setting of the patient monitor (20); and
a first communication section (13) configured to transmit a biological signal of a patient (P) acquired from a sensor attached to the patient (P) and the setting information that is input from the input section (12) to the patient monitor (20) when an instruction for transmitting setting information due to an operation on the display and input section (12) is issued;
wherein the communication section (13) is configured to receive a current setting status from the patient monitor (20), and
wherein the display section (12) is configured to display the setting screen for the patient monitor (20), the setting screen being based on the received setting status, and
wherein the patient monitor (20) comprises:
a second communication section (21) configured to receive the biological signal and the setting information from the input unit (10);
a second display (25) section configured to display vital sign information of the patient (P); and
a controller (22) configured to convert the biological signal to the vital sign information to control a display on the second display section (25), and to change, upon receipt of the setting information, the setting of the patient monitor (20) in a state in which the vital sign information is displayed on the second display section (25),
wherein the communication section (21) is configured to transmit a current setting status to the input unit (10); and
wherein the patient monitor (20) configured to change the setting in background.

## Patentansprüche

1. Eingabeeinheit (10), umfassend:
einen Anzeigebereich (12), der ausgelegt ist, um einen Einstellungsbildschirm anzuzeigen zum Ändern einer Einstellung eines mit der Eingabeeinheit (10) verbundenen Patientenmonitors (20);
einen Eingabebereich (12), der ausgelegt ist, um eine Eingabe von Einstellungsinformationen zu empfangen, die anzeigend sind für eine Steueranweisung zum Ändern der Einstellung des Patientenmonitors; und
einen Kommunikationsbereich (13), der ausgelegt ist, um ein biologisches Signal eines Patienten (P), das von einem Sensor erfasst wurde, der an dem Patienten (P) befestigt ist, an den Patientenmonitor (20) zu übertragen, um es zu erlauben, dass der Patientenmonitor (20) Lebenszeicheninformationen des Patienten (P) anzeigt, und um auch die Einstellungsinformationen, die über den Eingabebereich (12) eingegeben wurden, an den Patientenmonitor (20) zu übertragen, wenn eine Steueranweisung zum Übertragen von Einstellungsinformationen infolge einer Betätigung am Bildschirm und im Eingabebereich (12) ausgegeben wurde;
wobei der Kommunikationsbereich (13) ausgelegt ist, um einen aktuellen Einstellungsstatus von dem Patientenmonitor (20) zu empfangen, und
wobei der Anzeigebereich (12) ausgelegt ist, um den Einstellungsbildschirm für den Patientenmonitor (20) anzuzeigen, wobei der Einstellungsbildschirm auf dem empfangenen Einstellungsstatus beruht.

2. Eingabeeinheit (10) nach Anspruch 1, wobei der Anzeigebereich (12) und der Eingabebereich (12) in einen Berührungsbildschirm integriert sind.

3. Eingabeeinheit (10) nach Anspruch 1, weiter umfassend einen Speicherbereich (15), der ausgelegt ist, um den von dem Kommunikationsbereich (13) empfangenen Einstellungsstatus zu speichern.

4. Eingabeeinheit (10) nach Anspruch 2, wobei der Berührungsbildschirm ausgelegt ist, um einen Cursor anzuzeigen, und
wobei der Kommunikationsbereich (13) ausgelegt ist, um Informationen zu einer Betätigung des Cursors an den Patientenmonitor (20) zu übertragen.

5. Eingabeeinheit (10) nach Anspruch 4, wobei der Kommunikationsbereich (13) ausgelegt ist, um Koordinaten, zu denen der Cursor auf dem Berührungsbildschirm betätigt wird, an den Patientenmonitor (20) zu übertragen, und vom Patientenmonitor (20) Einstellungsbildschirminformationen zu empfangen, die den Koordinaten entsprechen, und
der Anzeigebereich (12) ausgelegt ist, um den Einstellungsbildschirm anzuzeigen, der den Einstellungsbildschirminformationen entspricht.

6. Eingabeeinheit (10) nach Anspruch 1, wobei der Kommunikationsbereich (13) ausgelegt ist, um die Einstellungsinformationen, die von dem Eingabebereich (12) eingegeben werden, an den Patientenmonitor (20) zu übertragen, um die Einstellung des Patientenmonitors (20) in einen Zustand zu ändern, in dem die Lebenszeicheninformationen auf dem Patientenmonitor (20) angezeigt werden.

7. Eingabeeinheit (10) nach Anspruch 1, weiter umfassend einen Speicherbereich (15), der ein Programm speichert, das den Einstellungsbildschirm für den Patientenmonitor (20) definiert.

8. Eingabeeinheit (10) nach Anspruch 1, wobei der Anzeigebereich (12) ausgelegt ist, um den Einstellungsbildschirm so anzuzeigen, dass aktuell eingestellte Werte von anderen Werten unterscheidbar sind.

9. Patientenmonitor (20), umfassend:
einen Kommunikationsbereich (21), der ausgelegt ist, um ein biologisches Signal eines Patienten (P) und Einstellungsinformationen von der Eingabeeinheit (10) nach Anspruch 1 zu empfangen, wobei die Einstellungsinformationen anzeigend sind für eine Steueranweisung zum Ändern einer Einstellung des Patientenmonitors (20);
einen Anzeigebereich (25) der ausgelegt ist, um Lebenszeicheninformationen des Patienten (P) anzuzeigen; und
eine Steuerung (22), die ausgelegt ist, um das biologische Signal in die Lebenszeicheninformationen umzuwandeln, um eine Anzeige in dem Anzeigebereich (25) zu steuern und bei Empfang der Einstellungsinformationen eine Einstellung des Patientenmonitors (20) in einen Zustand zu ändern, in dem die Lebenszeicheninformationen in dem Anzeigebereich (25) angezeigt werden, wobei der Kommunikationsbereich (21) ausgelegt ist, um einen aktuellen Einstellungsstatus an die Eingabeeinheit (10) zu übertragen;
wobei der Patientenmonitor (20) ausgelegt ist, um die Einstellung im Hintergrund zu ändern.

10. Patientenmonitor (20) nach Anspruch 9, wobei die Steuerung (22) bei Empfang einer Eingabe von Cursorbetätigungsinformationen von der Eingabeeinheit (10) einen Cursor an einer entsprechenden Position im Anzeigebereich (25) auf der Grundlage der Cursorbetätigungsinformationen anzeigt.

11. Patientenmonitor (20) nach einem der Ansprüche 9 bis 10, weiter umfassend einen Eingabebereich (25), der ausgelegt ist, um eine Eingabe von einem Bediener zu empfangen,
wobei die Steuerung (22) ausgelegt ist, um bei Empfang der Einstellungsinformationen von dem Eingabebereich (25) die Lebenszeicheninformationen und einen Einstellungsbildschirm auf überlagerte Weise in dem Anzeigebereich (25) anzuzeigen.

12. Patientenmonitor nach Anspruch 9, wobei der Anzeigebereich (25) ausgelegt ist, um die Lebenszeicheninformationen einschließlich einer Lebenszeichenwellenform und eines Messwerts anzuzeigen.

13. Patientenüberwachungssystem (1), umfassend eine Eingabeeinheit (10) und einen Patientenmonitor (20),
wobei die Eingabeeinheit (10) umfasst:
einen ersten Anzeigebereich (12), der ausgelegt ist, um einen Einstellungsbildschirm zum Ändern einer Einstellung des mit der Eingabeeinheit (10) verbundenen Patientenmonitors (20) anzuzeigen;
einen Eingabebereich (12), der ausgelegt ist, um eine Eingabe von Einstellungsinformationen zu empfangen, die anzeigend sind für eine Steueranweisung zum Ändern der Einstellung des Patientenmonitor (20); und
einen ersten Kommunikationsbereich (13), der ausgelegt ist, um ein biologisches Signal eines Patienten (P), das von einem Sensor erfasst wurde, der an dem Patienten (P) befestigt ist, und die Einstellungsinformationen, die über den Eingabebereich (12) eingegeben wurden, an den Patientenmonitor (20) zu übertragen, wenn eine Steueranweisung zum Übertragen von Einstellungsinformationen infolge einer Betätigung am Bildschirm und im Eingabebereich (12) ausgegeben wurde;
wobei der Kommunikationsbereich (13) ausgelegt ist, um einen aktuellen Einstellungsstatus von dem Patientenmonitor (20) zu empfangen, und
wobei der Anzeigebereich (12) ausgelegt ist, um den Einstellungsbildschirm für den Patientenmonitor (20) anzuzeigen, wobei der Einstellungsbildschirm auf dem empfangenen Einstellungsstatus beruht, und
wobei der Patientenmonitor (20) umfasst:
einen zweiten Kommunikationsbereich (21), der ausgelegt ist, um das biologische Signal und die Einstellungsinformationen von der Eingabeeinheit (10) zu empfangen;
einen zweiten Anzeigebereich (25), der ausgelegt ist, um Lebenszeicheninformationen des Patienten (P) anzuzeigen; und
eine Steuerung (22), die ausgelegt ist, um das biologische Signal in die Lebenszeicheninformationen umzuwandeln, um eine Anzeige in dem zweiten Anzeigebereich (25) zu steuern und bei Empfang der Einstellungsinformationen die Einstellung des Patientenmonitors (20) in einen Zustand zu ändern, in dem die Lebenszeicheninformationen in dem zweiten Anzeigebereich (25) angezeigt werden,
wobei der Kommunikationsbereich (21) ausgelegt ist, um einen aktuellen Einstellungsstatus an die Eingabeeinheit (10) zu übertragen; und
wobei der Patientenmonitor (20) ausgelegt ist, um die Einstellung im Hintergrund zu ändern.

## Revendications

1. Unité d'entrée (10) comprenant :
une section d'affichage (12) configurée pour afficher un écran de réglage pour modifier un réglage d'un système de surveillance de patient (20) raccordé à l'unité d'entrée (10) ;
une section d'entrée (12) configurée pour recevoir une entrée d'informations de réglage indicative d'une instruction de changement du réglage du système de surveillance de patient ; et
une section de communication (13) configurée pour transmettre un signal biologique d'un patient (P), acquis à partir d'un capteur attaché au patient (P), au système de surveillance de patient (20) pour permettre au système de surveillance de patient (20) d'afficher des informations relatives aux signes vitaux du patient (P) et également pour transmettre les informations de réglage qui sont entrées à partir de la section d'entrée (12) au système de surveillance de patient (20) lorsqu'une instruction de transmission d'informations de réglage due à une opération sur l'affichage et la section d'entrée (12) est émise ;
dans laquelle la section de communication (13) est configurée pour recevoir un statut de réglage actuel du système de surveillance de patient (20), et
dans laquelle la section d'affichage (12) est configurée pour afficher l'écran de réglage pour le système de surveillance de patient (20), l'écran d'affichage étant basé sur le statut de réglage reçu.

2. Unité d'entrée (10) selon la revendication 1, dans laquelle la section d'affichage (12) et la section d'entrée (12) sont intégrées en tant que panneau tactile.

3. Unité d'entrée (10) selon la revendication 1, comprenant en outre une section de stockage (15) configurée pour stocker le statut de réglage reçu par la section de communication (13).

4. Unité d'entrée (10) selon la revendication 2, dans laquelle le panneau tactile est configuré pour afficher un curseur, et
dans laquelle la section de communication (13) est configurée pour transmettre des informations sur un actionnement du curseur au système de surveillance de patient (20).

5. Unité d'entrée (10) selon la revendication 4, dans laquelle la section de communication (13) est configurée pour transmettre des coordonnées, selon lesquelles le curseur est actionné sur le panneau tactile, au système de surveillance de patient (20), et pour recevoir des informations d'écran de réglage correspondant aux coordonnées du système de surveillance de patient (20), et
la section d'affichage (12) est configurée pour afficher l'écran de réglage correspondant aux informations d'écran de réglage.

6. Unité d'entrée (10) selon la revendication 1, dans laquelle la section de communication (13) est configurée pour transmettre les informations de réglage qui sont entrées à partir de la section d'entrée (12) au système de surveillance de patient (20) pour modifier le réglage du système de surveillance de patient (20) dans un état dans lequel les informations relatives aux signes vitaux sont affichées sur le dispositif de surveillance de patient (20).

7. Unité d'entrée (10) selon la revendication 1, comprenant en outre une section de stockage (15) stockant un programme définissant l'écran de réglage pour le système de surveillance de patient (20).

8. Unité d'entrée (10) selon la revendication 1, dans laquelle la section d'affichage (12) est configurée pour afficher l'écran de réglage de telle sorte que des valeurs actuellement réglées peuvent être distinguées d'autres valeurs.

9. Système de surveillance de patient (20) comprenant :
une section de communication (21) configurée pour recevoir un signal biologique d'un patient (P) et des informations de réglage de l'unité d'entrée (10) selon la revendication 1, les informations de réglage étant indicatives d'une instruction de changement d'un réglage du système de surveillance de patient (20) ;
une section d'affichage (25) configurée pour afficher des informations relatives aux signes vitaux du patient (P) ; et
un système de commande (22) configuré pour convertir le signal biologique en informations relatives aux signes vitaux pour commander un affichage sur la section d'affichage (25), et pour modifier, à la réception des informations de réglage, un réglage du système de surveillance de patient (20) en un état dans lequel les informations relatives aux signes vitaux sont affichées sur la section d'affichage (25), dans lequel la section de communication (21) est configurée pour transmettre un statut de réglage actuel à l'unité d'entrée (10) ;
dans lequel le système de surveillance de patient (20) est configuré pour modifier le réglage en arrière-plan.

10. Système de surveillance de patient (20) selon la revendication 9, dans lequel le système de commande (22) affiche, à la réception d'une entrée d'informations d'actionnement de curseur de l'unité d'entrée (10), un curseur à une position correspondante sur la section d'affichage (25) sur la base des informations d'actionnement de curseur.

11. Système de surveillance de patient (20) selon l'une quelconque des revendications 9 à 10, comprenant en outre une section d'entrée (25) configurée pour recevoir une entrée d'un opérateur,
dans lequel le système de commande (22) est configuré pour afficher, à la réception des informations de réglage de la section d'entrée (25), les informations relatives aux signes vitaux et un écran de réglage de manière superposée sur la section d'affichage (25).

12. Système de surveillance de patient selon la revendication 9, dans lequel la section d'affichage (25) est configurée pour afficher les informations relatives aux signes vitaux incluant une forme d'onde des signes vitaux et une valeur de mesure.

13. Système de surveillance de patient (1) comprenant une unité d'entrée (10) et un système de surveillance de patient (20),
dans lequel l'unité d'entrée (10) comprend :
une première section d'affichage (12) configurée pour afficher un écran de réglage pour modifier un réglage du système de surveillance de patient (20) raccordé à l'unité d'entrée (10) ;
une section d'entrée (12) configurée pour recevoir une entrée d'informations de réglage indicative d'une instruction de changement du réglage du système de surveillance de patient (20) ; et
une première section de communication (13) configurée pour transmettre un signal biologique d'un patient (P) acquis à partir d'un capteur attaché au patient (P) et les informations de réglage qui sont entrées à partir de la section d'entrée (12) au système de surveillance de patient (20) lorsqu'une instruction de transmission d'informations de réglage due à une opération sur l'affichage et la section d'entrée (12) est émise ;
dans lequel la section de communication (13) est configurée pour recevoir un statut de réglage actuel du système de surveillance de patient (20), et
dans lequel la section d'affichage (12) est configurée pour afficher l'écran de réglage pour le système de surveillance de patient (20), l'écran d'affichage étant basé sur le statut de réglage reçu, et
dans lequel le système de surveillance de patient (20) comprend :
une seconde section de communication (21) configurée pour recevoir le signal biologique et les informations de réglage de l'unité d'entrée (10) ;
une seconde section d'affichage (25) configurée pour afficher des informations relatives aux signes vitaux du patient (P) ; et
un système de commande (22) configuré pour convertir le signal biologique en informations relatives aux signes vitaux pour commander un affichage sur la seconde section d'affichage (25), et pour modifier, à la réception des informations de réglage, le réglage du système de surveillance de patient (20) en un état dans lequel les informations relatives aux signes vitaux sont affichées sur la seconde section d'affichage (25),
dans lequel la section de communication (21) est configurée pour transmettre un statut de réglage actuel à l'unité d'entrée (10) ; et
dans lequel le système de surveillance de patient (20) est configuré pour modifier le réglage en arrière-plan.
